Europäisches Patentamt

**European Patent Office**

**Office européen des brevets**

(19)

(11) Publication number: **0 250 128**
A2

(12)

# EUROPEAN PATENT APPLICATION

(21) Application number: **87304908.4**

(22) Date of filing: **03.06.87**

(51) Int. Cl.⁴: **C 12 N 7/02,** C 12 N 5/00, G 01 N 33/569, A 61 K 39/21

(30) Priority: **18.06.86 US 875535**

(43) Date of publication of application: **23.12.87** Bulletin 87/52

(84) Designated Contracting States: **AT BE CH DE ES FR GB GR IT LI LU NL SE**

(71) Applicant: **American Registry of Pathology, Armed Forces Institute of Pathology, Washington, D.C. 20306 (US)**

(72) Inventor: **Lo, Shyh-Ching, 4804 Cloister Drive, Rockville Maryland 20852 (US)**

(74) Representative: **Perry, Robert Edward et al, GILL JENNINGS & EVERY 53-64 Chancery Lane, London WC2A 1HN (GB)**

(54) **Novel virus isolated from patients with aids.**

(57) The invention relates to a novel virus isolated from patients with Acquired Immune Deficiency Syndrome (AIDS) and to its use in detecting antibodies in sera of AIDS patients and patients with AIDS-related complex (ARC), and to its use as a vaccine against infection by the virus. The invention further relates to antibodies against the virus and their use in detecting viral antigens in infected tissue of patients with AIDS.

EP 0 250 128 A2

## TITLE OF THE INVENTION
NOVEL VIRUS ISOLATED FROM PATIENTS WITH AIDS

The invention described herein was made in the course of work under a grant or award from the United States Department of the Army.

## BACKGROUND OF THE INVENTION

### Field of the Invention

The present invention relates to a novel virus isolated from patients with Acquired Immune Deficiency Syndrome (AIDS) and to its use in detecting antibodies in sera of AIDS and AIDS-related complex (ARC), sometimes referred to as pre-AIDS, patients, and its use as a vaccine against infection by the virus. The invention further relates to antibodies against the virus which are useful in detecting viral antigens in infected tissue of AIDS patients.

### Description of the Prior Art

Acquired Immune Deficiency Syndrome (AIDS) is a devastating new disease that has afflicted over 20,000 people worldwide (AIDS Weekly Surveillance Report - United States, Centers for Disease Control, May 5, 1986). The disease is clinically characterized by the development of opportunistic infections and certain uncommon malignancies like Kaposi's sarcoma or B-cell lymphoma (Durack, D.T., N.Eng.J.Med. 305, 1465 (1981); Reichert, C.M., et al., Am.J.Path. 112, 357 (1983); Ziegler, J.L., et al., N.Eng.J.Med. 311, 565 (1984). Although a viral etiology of this disease has long been suggested, conventional approaches for isolating infectious viral agents have not been fruitful. Through co-cultivation of AIDS patients' peripheral blood cells with mitogen-stimulated normal human lymphocytes or permanent human T-cell lines, a number of laboratories have isolated T-cell-tropic human retroviruses (HTLV-III/LAV), Barre-Sinoussi, F., et al., Science 220, 868 (1983); Gallo, R.C., et al., Science 224, 500 (1984). Epidemiologically, these newly isolated retroviruses have been

0250128

-2-

shown to be highly associated with patients of AIDS and/or AIDS-related complex (ARC). Schupbach, J., et al., _Science_ 224, 503 (1984); Sarngadharan, M.G., et al., _Science_ 224, 506 (1984). In _vitro_ studies with HTLV-III/LAV have demonstrated T-cell tropism and cytopathic changes. Barre-Sinoussi, F., et al, _supra_; Popovic, M., et al., _Science_ 224, 497 (1984). However, the establishment of an animal model of AIDS by HTLV-III/LAV injection has not been successful. Gajdusek, D.C., et al., _Lancet_ I, 1415 (1984). The chimpanzee is the only primate other than man found to be susceptible to infection by HTLV-III/LAV. However, overt AIDS manifested by the development of opportunistic infections and/or unusual malignancies has not yet been seen, despite evidence for persistent infection and/or viremia in experiments on this species. Gajdusek, D.C., et al., _Lancet_ I, 55 (1985). Thus, the human retroviruses have not fulfilled Koch's postulates, i.e., producing transmissible AIDS-like diseases in experimental animals.

## SUMMARY OF THE INVENTION

The present invention relates to a novel virus isolated from patients with AIDS. The invention further relates to the use of the virus for the detection of antibodies in sera of AIDS patients and patients with ARC, and for vaccines against the novel virus. The invention also relates to antibodies to the virus and their use in detecting viral antigens in the tissue of AIDS patients.

The virus is isolated from the spleen of patients with AIDS or from Kaposi's sarcoma tissue from patients with AIDS. The virus is capable of transfecting and transforming NIH/3T3 cells. The DNA of the transformants does not contain human repetitive DNA sequences. Two transformants are identified as Sb51 and Kb43. These transformants are persistently infected by a virus. A large quantity of viral nucleocapsids are located in many of the nuclei of the transformed cells. The virus is isolated from the transformants. The majority of mature virions have a size of about 140 nm to about 280 nm, with an overall range of 100-900 nm. Introduction of the virus into nude mice and immunocompetent mice (Balb/c) results in a significant morbidity and mortality of the infected animals and the manifestation of many symptoms similarly seen in patients with AIDS. Similar diseases are transmitted from animal to animal by introduction of infected tissues. The novel virus is capable of detecting antibodies in sera of patients with AIDS or ARC. Any method for detecting an antigen-antibody reaction may be utilized, including enzyme-linked immunosorbent assay (ELISA), immunoradiometric assay, direct and indirect immunofluorescent assay, Western blot technique, and the like. In addition, antibodies are raised in experimental animals or monoclonal antibodies against the virus which are capable of detecting viral-related antigens in infected tissues, including peripheral blood cells and sera of patients with AIDS.

Since antibodies are successfully raised against the virus, the virus or viral antigens can be utilized to prepare vaccines which will protect animals, including humans, against infection by the virus.

BRIEF DESCRIPTION OF THE DRAWINGS

Figure 1 shows (A) a transformed colony transfected by nucleic acid isolated from splenic tissue of a patient with AIDS, and (B) monolayers of the transformed cells.

Figure 2 shows (A) monolayers of Sb51 cells with little evidence of cytopathic changes, (B) and (E) cytopathic changes in the center of high cell density foci, (C) and (F) a cytolytic plaque, and (D) formation of high cell density foci and accumulation of multiple cell layers in Sb51 monolayers.

Figure 3 shows assemblage of nucleocapsids in NIH/3T3 cells persistently infected with the virus. The nucleocapsids are seen within the nucleus, (A), (B), (C), within membrane-bound cisternae in cytoplasm (D), and within cytoplasm free of membrane (E).

Figure 4 shows ultrastructural studies of viral maturation in cytolytic Sb51 cells in nucleus (A) and cytoplasm (B). Mature virions in the cytoplasm of disrupted Sb51 cells is shown in (C), (D) and (E).

Figure 5 shows viral particles released in the culture supernatant of Sb51 cells including membrane-bound viral particles (A), an intact particle (B), and a partially disrupted virion (B) and (C).

Figure 6 shows immunocytochemical staining of (A) Sb51 cells with non-AIDS serum, (B) NIH/3T3 cells with AIDS serum, and (C) Sb51 cells with AIDS serum.

## DETAILED DESCRIPTION OF THE INVENTION

The invention relates to a novel virus isolated from patients with AIDS. A virus according to the invention, in persistently infected cells, is deposited with the American Type Culture Collection under Deposit No. CRL 9127, deposited on June 17, 1986. The virus is isolated from tissues of AIDS patients, such as Kaposi's sarcoma tissue or splenic tissue. The tissue is minced into small pieces and treated with collagenase. The tissue suspension is then treated with a proteinase, such as proteinase K. Genetic materials are obtained after phenol extraction, phenol/chloroform/isoamylalcohol extraction, and chloroform/isoamylalcohol extraction. High molecular weight DNA is visibly observed after ethanol precipitation of the genetic materials. The genetic materials are dissolved and contain high molecular weight DNA and RNA of various sizes.

The isolated genetic materials are utilized to transfect NIH/3T3 cells in accordance with the procedure of Graham, F.L., et al., Virology 52, 456 (1973). In this procedure, the nucleic acid is precipitated with calcium phosphate and incubated with NIH/3T3 cells. The precipitated nucleic acid is removed and the cells trypsinized. The trypsinized cells are reseeded and treated with glycerol before splitting, as described by Copeland, N.G., et al., Cell 16, 347 (1979). The subcultures are fed with Dulbecco's medium with fetal bovine serum (FBS) and refed at three- to four-day intervals. Foci of morphologically transformed cells become evident in about two weeks. The phenotypical transformation is characterized by rapid overgrowth of the transfected cells which pile up in multilayers and form grossly visible foci. Transformation efficiency is about 0.01-0.02 identifiable foci per microgram of donor nucleic acid. Transformed colonies are harvested after three weeks, and are cultured in monolayers. The DNA of the transformants does not contain human repetitive DNA sequences.

Genetic materials are isolated from the primary transfectants as previously described, and used to transfect fresh NIH/3T3 cells. Transformation is again seen using the genetic materials with a slightly higher transformation efficiency. This demonstrates that the genetic materials isolated from tissues of AIDS patients contain active transforming elements.

Most of the virions are found to be cell-associated. The virus is isolated from the transformants, such as Sb51. In general, Sb51 cell pellets are lysed by freezing and thawing to release the viral particles. The large viral particles are pelleted through a sucrose barrier and banded in a sucrose isopycnic gradient. The intact viral particles have a density of about 1.17 to about 1.20. The primary genetic material isolated at this density appears to be RNA.

The novel virus of the invention is characterized by mature virions having a wide range of size (100-900 nm) with the majority of the virions having a size of about 140 nm to about 280 nm.

The novel virus of the invention can be introduced into mice. In general, the virus isolated from $5 \times 10^6$ Sb51 cells is injected either intravenously or intraperitoneally into a six-week-old mouse. Nude mice or immunocompetent mice (Balb/c) can be infected. Infection of nude mice with the novel virus of the invention results in significant mortality of the infected animals. Many symptoms similarly seen in patients with AIDS are induced in the infected mice. Thus, at necropsy, the infected mice often showed prominent systemic lymphadenopathy, neuropathy or lymphoid depletion with varying degrees of plasmacytosis. Signs of immune deficiency with profound cutaneous infection in some of the animals are noted.

-6-

Disseminated pruritic skin rashes are also common. There are proliferative lesions of spindle cells in the cutaneous tissue and deep viscera. The immunocompetent mice (Balb/c) infected by the virus are found to be subsequently infected by Pneumocystis carinii, which is evidence of the immunodeficient state of these infected animals.

Similar diseases are transmitted from animal to animal by injecting filtrated lysates of spleen, lymph nodes or whole blood from the diseased animals. Massive accumulation of viral nucleocapsids is seen in the cellular nuclei of infected tissues. Mature virions are also identified in the cytoplasm of the cytopathic cells.

The novel virus of the invention is useful for the detection of antibodies in the sera of patients with AIDS or ARC. In one procedure, persistently virus-infected cells are grown in low cell density on sterile glass slides. Sera from AIDS patients, patients with ARC, and normal subjects are examined in an immunoperoxidase staining procedure such as described by Hsu, S-M., et al., Am.J.Clin.Path. 80, 21 (1983). Using this assay, 23 of 24 sera from AIDS patients showed strong positivity. Serum from the other AIDS patient showed weak positivity. Twenty-six of 30 sera from normal subjects showed no reactivity with the persistently infected cells (Sb51). Four of 30 sera from normal subjects showed mild reactivity (weak positivity), but the staining was significantly less than the staining seen in the reactivity with sera of AIDS patients. Sera examined from all patients with ARC showed strong positivity, i.e., the sera contained antibodies which reacted with Sb51 cells. In addition, some of the sera from infected animals, as described above, also contained antibodies which reacted with the persistently viral infected cells in this assay procedure.

In addition to this procedure, any other procedure for detecting an antigen-antibody reaction can be utilized to detect antibodies to the novel virus of the invention in the sera of AIDS patients or patients with ARC. Such procedures include, but are not limited to, ELISA, Western-blot, direct or indirect immunofluorescent assay and immunoradiometric assay. Such assay procedures for the detection of antibodies in sera of AIDS patients or patients with ARC have been described in U.S. Patent 4,520,113, incorporated herein by reference, which uses HTLV-III/LAV as the antigen. Similar procedures employing the virus of the present invention can be used. A diagnostic kit for the detection of AIDS-specific antibodies can be prepared in a conventional manner using the virus of the invention. It is expected that assays utilizing these techniques, especially Western-blot, will provide better results, particularly fewer false-positives.

Antibodies against the virus of the invention are produced in experimental animals such as mice, rabbits and goats, using standard techniques. Alternatively, monoclonal antibodies against the virus can be prepared in a conventional manner. The antibodies are useful for detecting viral antigens in infected tissues such as lymph nodes, spleen, Kaposi's sarcoma, brain and peripheral blood cells, as well as sera, of patients with AIDS. Any procedure useful for detecting an antigen-antibody reaction, such as those described above, can be utilized to detect the viral antigens in tissues of patients with AIDS.

The antibodies are also useful for detecting cells which have been infected by the virus. This capability is useful for the isolation of the virus from other tissues. For example, additional virus can be isolated by co-cultivating infected tissue from patients with AIDS and a suitable recipient cell line or cells, such as lymphocytes. The infected cells are assayed or recognized by the antibody, and the virus can be obtained from the infected cells as described above. An affinity column can also be prepared using the antibodies and used to purify the virus from the infected cell lysate.

The virus or viral antigens can be utilized as a vaccine in a conventional manner to induce the formation of protective antibodies. The viral antigens can be isolated from the virus or can be produced by conventional recombinant DNA techniques. The vaccines are prepared by usual procedures, such as by in

_vitro_ cell cultures, by recombinant DNA techniques, and by application of the usual and prescribed controls to eliminate bacterial and/or viral contaminations, according to well known principles and international standard requirements.

Preferably an inactivated, i.e., attenuated or killed, vaccine is utilized. The virus is isolated from the infected cells grown in monolayers. The virus is killed by known procedures or modifications thereof, e.g., by the addition of beta-propiolactone, Formalin or acetylethyleneimine, by ultraviolet radiation, or by treatment with psoralen or psoralen derivatives and long-wavelength ultraviolet light. Alternatively, the virus is attenuated by conventional techniques and isolated. The vaccine of the invention may contain one or more suitable stabilizers, preservatives, buffering salts and adjuvants. The vaccine may be formulated for oral or parenteral administration. Compositions in the form of an injectable solution may contain a proper titre of the virus as the active ingredient, and may also contain one or more of a pH adjuster, buffer, stabilizer, excipient and an additive for rendering the solutions isotonic. The injectable solutions may be prepared to be adapted for subcutaneous, intramuscular or intravenous injection by conventional techniques. If desired, the solutions may be lyophilized in a usual manner to prepare lyophilized injections.

Solid compositions for oral administration, which may be in the form of tablets, coated tablets, granules, powders and capsules, may contain excipients for the active ingredients and, if required, other additives including binders, disintegrators, lubricants, colorants, sweetening agents and flavorings.

The dosage of the virus administered will, of course, depend on the mode of administration and the interval of administration. An optimal dose of the active ingredient and an optimal interval of administration can easily be determined by routine preliminary tests known in the art.

The present invention is further illustrated by reference to the following examples. These examples are provided for illustrative purposes, and are in no way intended to limit the scope of the invention.

## EXAMPLE 1

### Isolation of Genetic Materials from AIDS Patients

Kaposi's sarcoma tissue was obtained from a patient with AIDS who died of fulminant Pneumocystis carinii pneumonitis. At autopsy, extensive Kaposi's sarcoma involving skin, both lungs, parietal pleura, gastrointestinal tract, pancreas, liver, kidney and lymph nodes was found. The tissue used to extract genetic material was derived from Kaposi's sarcoma in the patient's retroperitoneal lymph nodes, five to six hours after death. Permanent paraffin sections confirmed near-total effacement of lymph node architecture by Kaposi's sarcoma.

Splenic tissue was obtained from a second patient with AIDS who also died of P. carinii pneumonitis. No tumor (i.e., Kaposi's sarcoma or lymphoma) was identified at autopsy. Paraffin sections of the splenic tissue used to extract genetic material showed congestion and lymphocyte depletion.

The splenic or Kaposi's sarcoma tissue (1-2 g) was minced into small pieces and treated with collagenase (5 mg/ml) in 1 ml phosphate-buffered saline (PBS) at 37°C for 15 minutes. The tissue suspension was then treated with proteinase K (250 μg/ml) in 10X volume of 150 mM NaCl, 10 mM Tris (pH 7.5), 0.4% SDS, at 65°C for 30 minutes and at 37°C for ten hours. Phenol extraction (twice) followed by phenol/chloroform/isoamylalcohol (25:24:1) and chloroform/isoamylalcohol (24:1) extraction were used to purify genetic material. Grossly visible high molecular weight DNA was easily observed after ethanol precipitation. The genetic materials were redissolved in aqueous phase (1 mM Tris, 1 mM EDTA) after overnight air-drying. The recovered genetic materials contained high molecular weight DNA and 30-40% RNA of various size. The procedures of isolating genetic materials from the cultures of the primary transformants and normal human fibroblasts (ATCC,

-10-

CRL-1521) were similar. The pellets of 10-20 x $10^6$ cells were mixed directly with 10X volume of proteinase K (250 µg/ml) in the same buffer without collagenase treatment.

## EXAMPLE 2

### Transfection of NIH/3T3 Cells

The transfection procedures were slightly modified from that of Graham et al., supra. Approximately 30 micrograms of nucleic acid isolated from Kaposi's sarcoma tissue, splenic tissue, normal human fibroblast, or salmon sperm were precipitated with calcium phosphate in each 60 mm Petri dish culture (containing about 5 x $10^5$ NIH/3T3 cells). The DNA precipitate was removed after cells were incubated at 37°C for 12 hours. After an additional 24 hours, each plate of cells was trypsinized and reseeded into four to five 60 mm Petri dishes. The cells received five minutes of 15% glycerol treatment in 10% fetal bovine serum (FBS, Gibco) Dulbecco's modified Eagle's medium (DMEM) before the splitting as described by Copeland et al., supra. The subcultures were fed with Dulbecco's medium with 5% FBS and re-fed with this medium at intervals of three to four days. Foci of morphologically transformed cells became evident in two weeks. Colonies were harvested after three weeks.

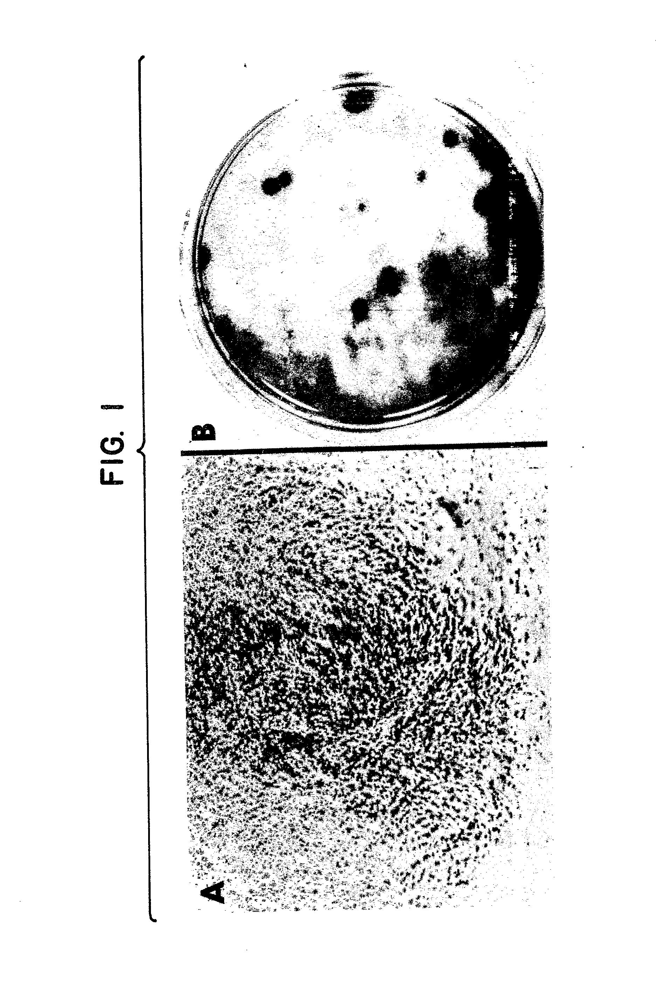

NIH/3T3 cells transfected with genetic material derived from both spleen and Kaposi's sarcoma tissue of AIDS patients produced morphologically transformed colonies which were visible within two weeks. The phenotypical transformation was characterized by the rapid overgrowth of the transfected cells which piled up in multilayers and formed grossly visible foci (Figure 1(A) at 75X magnification). Transformation efficiency was approximately 0.01 to 0.02 identifiable foci per microgram of donor nucleic acid. In contrast, no transformed foci were identified in parallel cultures using DNA from salmon sperm or nucleic acid from human fibroblasts. The transformants were recovered from these phenotypically malignant foci after two weeks and cultured in

-11-

monolayers. Transformants retained their tendency of piling up in multi-layers and reached more than three-fold higher cellular denisty than normal NIH/3T3 fibroblasts. One of the transformants grown in monolayers is shown in Figure 1(B) to demonstrate the prominence of forming random multilayers and high cell density foci in these cultures.

## EXAMPLE 3

### Confirmation of NIH/3T3 Cell Transformation

To confirm that transformation of the NIH/3T3 cells was mediated by active transforming genetic elements, the primary transformants' capacity to transmit their malignant phenotypes of rapid cell growth and pile-up in high cellular density in subsequent cycles of transfection was examined. Thus, a second cycle of transfection, as described above, was performed using genetic material which was isolated as previously described from some of the primary transfectants. A higher efficiency of transformation was observed in the second cycle of the transfection assay (up to 0.05 foci per microgram of donor nucleic acid). These results indicate that genetic materials isolated from spleen and Kaposi's sarcoma tissues of the AIDS patients contained active transforming elements that induce malignant transformation of rapid cell growth upon transfection and retransfection of phenotypically normal cells. The active transforming genetic elements have been similarly passed through four cycles of transfection. DNA from first and second stages of transformation clones selected for further studies were then characterized with respect to the presence of human DNA repetitive sequences by probing with $P^{32}$ nick-translated Blur 8-plasmid. No human repetitive DNA sequences were detected in these transformants.

-12-

EXAMPLE 4

Analysis of Transformants

Normal NIH/3T3 and transformant clones were all routinely maintained in monolayer cultures with 10% FBS-supplemented Dulbecco's media. Autoclavable slides (Cell-Line Asso. Inc.) were previously sterilized and overlayed with trypsinized cell suspension $(1 \times 10^5$ cells/ml) in square petri dishes. The cultures were incubated at 37°C in a 5% $CO_2$ incubator for 48 to 72 hours. The culture slides were washed three times with cold PBS, air-dried and stored at 4°C. Immunocytochemistry was performed within two to three days on these stored slides.

The monolayers were scraped directly from the cultures. The cells were harvested by centrifugation at 1,000 rpm for 10 minutes. The cell pellets were fixed overnight at 4°C in 2.5% glutaldehyde in phosphate buffer and post-fixed with 1% $OsO_4$. The fixed tissues were then processed by standard methods and embedded in Maraglass 655. The grids with ultra-thin sections were double-stained with uranylacetate and lead citrate. The specimens were then examined under an electron microscope with 60 kv or 100 kv voltage. Negative staining of the viral particles in the culture supernatants was performed. Briefly, the particles in the culture supernatant were pelleted through a 5 ml 20% sucrose barrier in SW41 centrifugation tubes, at 40,000 rpm for one hour. The pellets were then resuspended in 1/50 to 1/100 volume of Tris-normal saline (pH 7.4, 0.05 M Tris). The suspensions were directly put on formvar coated grids and negatively stained with 2% phosphotungstic acid (PTA) (pH 7.2).

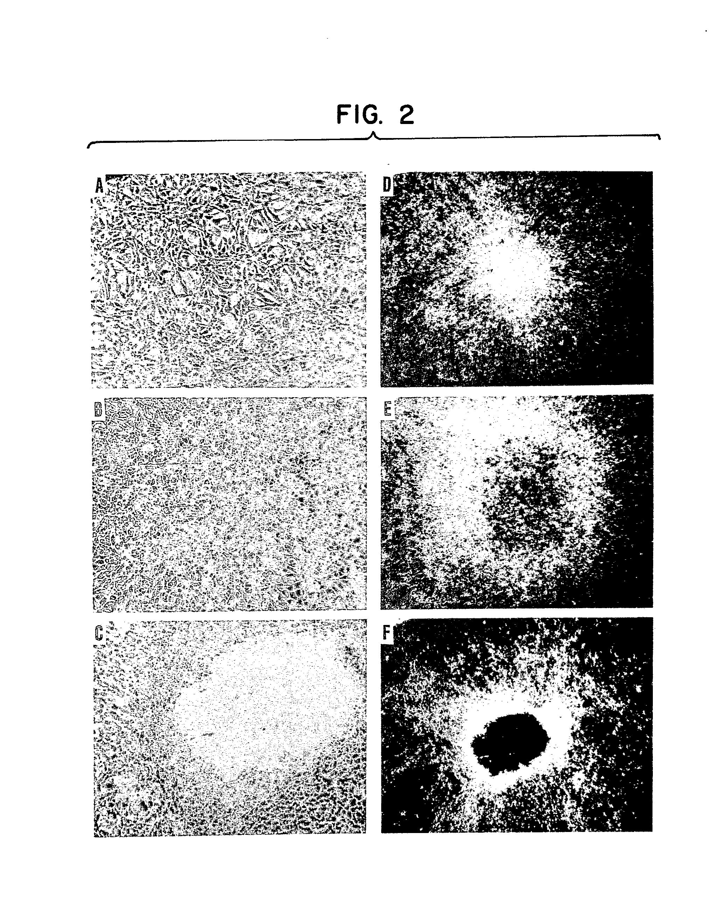

Two of the transformants (Sb51 and Kb43) were studied in detail. These two transformants were obtained from the second cycle transfections with genetic materials from spleen and Kaposi's sarcoma tissues, respectively. Sb51 cells persistently infected with the virus were deposited with the ATCC under No. CRL 9127 under the Budapest Treaty on June 17, 1986. They grew in high cellular density with no significant cytopathic changes (Figure 2(A) at 100X magnification). However, occasional lytic plaques, with cells showing cytopathic changes, were noted after the transformants reached saturated density. Many physiologic factors, including incubation temperatures and culture media, were found to affect the degrees of lytic plaque formation. For example, a reduction in the temperature to 32°C results in higher lytic plaque formation. Figure 2 depicts the pile-up of Sb51 cells in a monolayer culture. Foci of rapid cell overgrowth and pile-up into multicellular layers can best be appreciated under low-power light microscopy with a dark background (Figure 2(D) at 40X magnification). Cytopathic changes commonly occurred in the centers of the high cell density foci (Figure 2(B) at 100X magnification and Figure 2(E) at 40X magnification), and would lead to lytic plaques with apparently sclerotic edges (Figure 2(F) at 40X magnification). The same plaque is also shown under phase microscopy with a higher magnification (Figure 2(C) at 250X magnification). Detachment of the cytolytic cells in the center of hyperplastic foci was evident. There were prominent cytopathic effects among the densely-packed cells on the peripheral edges of the lytic plaque. These cells rounded up and appeared smaller in size with a shrunken configuration.

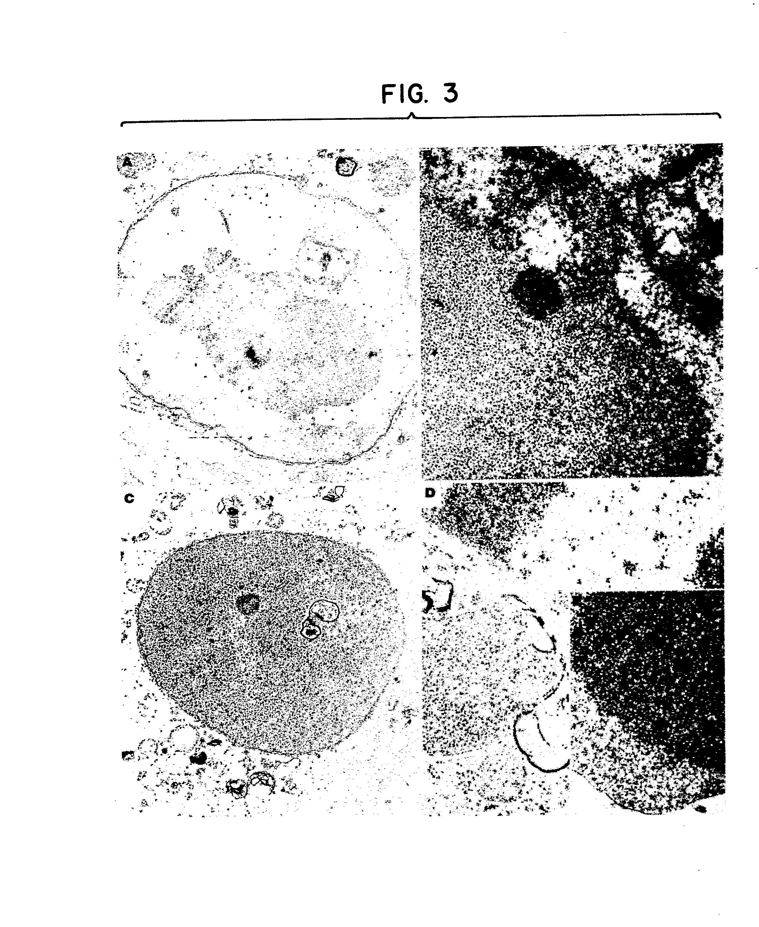

The monolayers of Sb51 and Kb43 which showed significant cytopathic changes in at least 30% of cells were examined by electron microscopy. Many transfectants showed evidence of a buildup of a large quantity of nucleocapsids in nuclei and some cytoplasms. Only a small portion of this material appeared to be used to form mature virions. The nucleocapsids were 8-9 nm thin tubules with fuzzy external coats, probably formed by assembly of rich nucleocapsid proteins. They were comonly organized into more granular configurations (about 18 nm to 25 nm in diameter) with much higher electron denisty (Figure 3). Viral nucleocapsids were found in the nuclei of transformed cells (Figure 3(A) at 13,400X magnification and Figure 3(B) at 47,000X magnification). Figure 3(C) at 14,700X magnification shows a cytolytic cell whose entire nucleus has been replaced by viral nucleocapsids. In this instance, few mature virions were seen in the cytoplasm. The

-14-

large cytoplasmic viroplasts containing viral genetic material were often locally concentrated and found to be membrane-bound (Figure 3(D) at 52,200X magnification). Early formation of this type of viroplast appeared in the cisternae of endoplasmic reticulum, which were markedly inflated and often carried ribosomes on their external membrane. There were occasional mature virions in the vesicles and endoplasmic reticulum cisternae. Nucleocapsids also formed in the cytoplasm without association of membrane (Figure 3(E) at 52,200X magnification) and were morphologically indistinguishable from nuclear nucleocapsids.

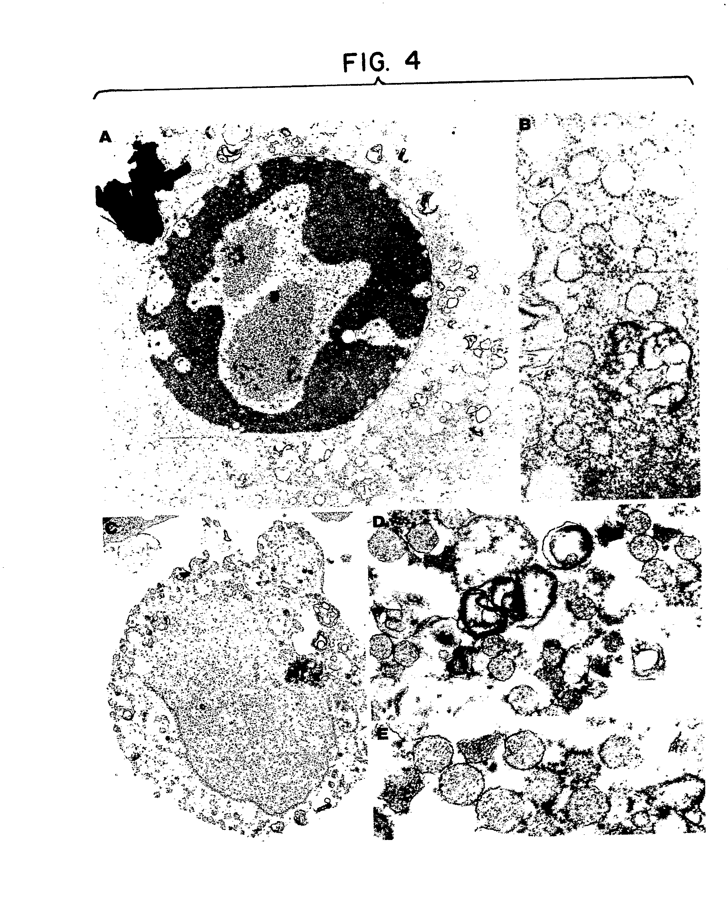

In those cells undergoing lytic cytopathic changes numerous virions were seen, mainly in the cytoplasm of disrupted cells (Figure 4). Early cytopathic changes showing nuclear chromation condensation and margination is seen in Figure 4(A) at 15,000X magnification. Accumulation of viral nucleocapsids within the nucleus is prominent. Numerous viral particles of different maturation stages were seen in the cytoplasm. This feature is also shown in Figure 4(B) at 45,800X magnification. However, occasional mature virions were also identified in the nuclei among the matrix of viral nucleocapsids. Most of the mature virions in the cytoplasm are lined up along the plasma membrane while others are free (Figure 4(C) at 12,800X magnification, Figure 4(D) at 45,800X magnification, and Figure 4(E) at 76,700X magnification). The virions were roughly spherical enveloped particles of heterogenous sizes. The majority of mature virions were 140-280 nm, with an overall range of 100-900 nm. The intact viral particle had a well-defined outer limited membrane about 8 nm thick and tightly packed internal nucleocapsids. Occasionally, ·the nucleocapsids were seen to condense into compact cores inside the virion. Although the virion's outer evelope was well-defined and thick, it was not rigid. Elongated, ovoid, and pleomorphic forms with protrusions were not uncommonly identified among the virions (Figure 4(D) at 45,800X magnification).

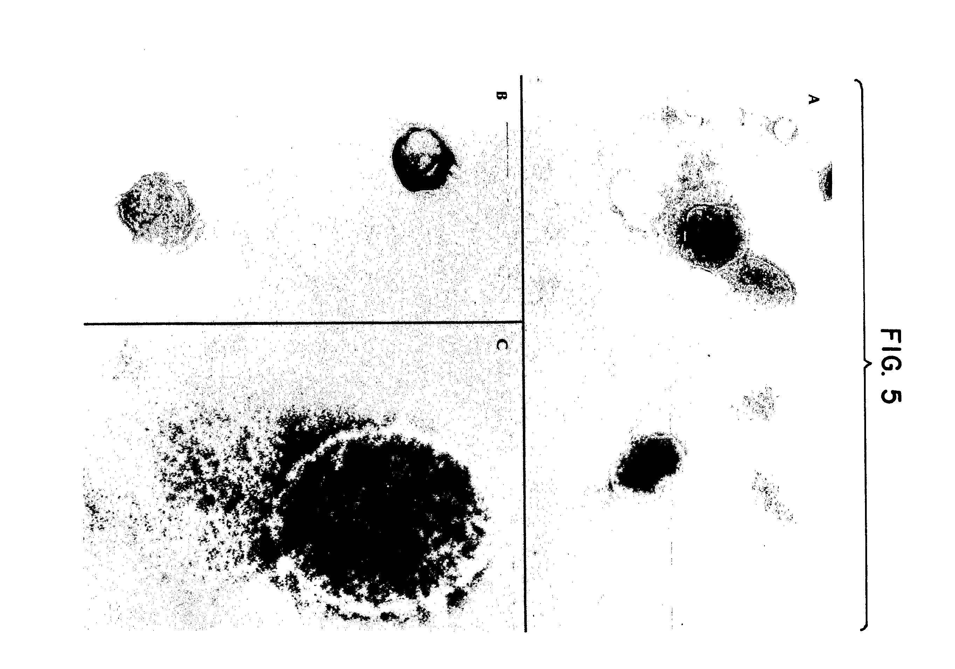

To further confirm the ultrastructure and morphology of the virus, the unsectioned virions were examined by pelleting viral particles from Sb51 and Kb43 culture supernatants through a 20% sucrose gradient barrier. The particles were resuspended in Tris-normal saline at 1/100 of original volume. The precipitated particles were directly examined under electron microscopy following negative stainings with PTA. Some preparations of the intact viral particles were briefly fixed with 0.5% Formalin to preserve the virions' morphology as well as to avoid possible infectious problems in the laboratory. The negative staining preparations of the virions usually revealed more surface detail together with their internal structure. A typical preparation of roughly spherical virions is shown in Figure 5. There was some heterogeneity in both particle size and shape. Some virions often appeared to be elongated or had irregular bulging protrusions (Figure 5(A) at 101,800X magnification). The internal component consisted of strands arranged more or less parallel to each other and to the long axis of the particle. The internal nucleocapsid strands appeared to be better preserved in the particles fixed with low concentrations of Formalin (lower particle, Figure 5(B) at 40,000X magnification). The broken outer membrane shown in the lower particle in Figure 5(B) was flipped upward (arrow heads). The well-defined envelope revealed inconspicuous spikes on the external surface (upper particle, Figure 5(B)). At high magnification, the virions demonstrated complex membranous envelopes (Figure 5(C) at 370,000X magnification). The released nucleocapsids appear to be uncoiled.

## EXAMPLE 5

### Isolation of Infectious Virus

Sb51 cells grown as monolayers were briefly trypsinized and pelleted by centrifugation at 1,000 rpm for 10 minutes. The cell pellet was resuspended with an equal volume of Dulbecco's medium. The cells were lysed by five cycles of freezing and thawing to release the cell-associated viral particles. The particles were pelleted through a 20% sucrose barrier in a SW41 centrifuge tube by centrifugation at 40,000 rpm for 45 minuts. The particles were

-16-

resuspended in PBS and banded in a sucrose isopycnic gradient (20% to 60%). The viral particles were localized at a density of about 1.17 to about 1.20. The viral particles were directly identified by electron microscopy with PTA negative staining. Those viral particles isolated from the fractions of the specified densities were then used for in vitro and in vivo infectivity studies. In the in vitro infectivity studies, cultured human embryo cells were directly infected with the isolated virus. The cells were assayed with antisera produced in Example 6. It was found that the cells were infected by the virus.

## EXAMPLE 6

### Production of Antibodies Against the Novel Virus

Viral particles were isolated as described in Example 5 from $5 \times 10^6$ Sb51 cells, and mixed with Freunds adjuvant. Rabbits were injected with the immunogen twice at a two- to three-month interval. A good antibody response to the virus was obtained after the second immunization.

## EXAMPLE 7

### Infection of Mice by the Novel Virus

The virus was isolated as described in Example 5 from $5 \times 10^6$ Sb51 cells, and resuspended in a small amount of PBS. The virus suspension was injected into either a six-week-old NIH (Nu Nu) male mouse or a six-week-old Balb/c male mouse. The injection was performed either intravenously or intraperitoneally. Sixty percent of the nude mice who received intravenous or intraperitoneal injections of viral preparation showed evidence of skin rashes with areas of erythematous changes and conjuctivitis in 10 to 12 days. One animal also showed prominent periorbital edema. These signs disappeared after two to three weeks. All the animals appeared to recover from the acute infection. Two animals then developed pruritic skin rashes after six weeks. These two animals and the other two animals died or became too sick, and had to be sacrificed in three months. Therefore, 40% of the animals had died in the first three months following viral injection. One animal which did not develop recognizable skin lesions showed systemic lymphadenopathy and paralysis. The animal appeared to be wasting and experienced complete paralysis of its hind legs. One animal had several purplish skin lesions which were slightly raised. At necropsy, all lymph nodes in these animals showed lymphocyte depletion. Only very small lymph nodes were identified on gross examination. In contrast, disseminated lymphadenopathy was seen in one of the animals. Markedly enlarged lymph nodes were seen in inguinal, axillary, cervical, mediastihal and mesentary lymph nodes. The animal also developed hepatosplenomegaly. Histologic sections of the lymph nodes revealed prominent plasmacytosis. Areas of sinus histiocytosis were also noted. The plasma cells effaced normal lymph node architecture and diffusely infiltrated the sinus. Lymph nodes in all the other animals showed lymphocyte depletion. Only small lymph nodes could be identified grossly.

Histologic sections of the purplish skin lesions revealed spindle cell proliferation. The spindle cells appeared to infiltrate cutaneous adipose tissue as well as underlying muscles. Extravasation of red blood cells was seen in some areas. Miototic figures were identified, but not prominent. Histologic examination of the liver of the animal also revealed spindle cell proliferation in the periportal areas. The homogeneous tumor cells exhibited more epithelioid appearance. Numerous red blood cells were trapped in the intercellular slits. Electron microscopic examiantion of the infiltrating spindle cells in the skin lesions revealed cells with cytopathic changes. An accumulation of viral nucleocapsids were seen in many of the nuclei, and some in the cytoplasm. The morphology and the characters of these viral nucleocapsids were similar to those observed in Sb51 cells previously described. Mature virions were also identified in some of the disrupted cells. Both nucleocapsids and virions were often seen in dilated cisternae

-18-

of smooth endoplasmic reticulum. Electron microscope studies of the periportal spindle cell lesions in the liver similarly revealed prominent infection of the virus.

Balb/c mice infected with the virus also appeared to be sensitive to the viral pathogen. Three of seven animals died in the first three months following infection. Two more animals died in the fourth month following infection. None of the control animals showed any disease in four months. Clinical evaluation of skin rashes and lymphadenopathy while these animals were alive was much more difficult. At necropsy, all of the animals were found to be lymphocyte-depleted. The animals had very small lymph nodes and spleens. Lymph nodes were often unrecognizable grossly. The lungs of these animals were found to have severe pneumonitis. M-Ag and tolulent blue staining revealed P. carinii. Therefore, these animals were believed to be severely immunodeficient. Two of the animals who survived for more than four months were found to have antibody in their sera which recognized Sb51 cells but not NIH/3T3 parental cells. Immunoperoxidase reaction of the sera showed positive reactions in both the nuclei and the cytoplasm of Sb51 cells.

EXAMPLE 8

Detection of Antibodies Against Novel Virus

Sera from AIDS patients and from normal subjects were analyzed by the immunoperoxidase straining procedure as described by Hsu et al., supra. Briefly, persistently infected Sb51 cells or normal NIH/3T3 cells were grown in low cell density on sterile glass slides. The culture slides were fixed in acetone at room temperature for five minutes. After washing in Tris-buffered saline (TBS), pH 7.6, 0.05 M, the slides were first incubated with 1% normal horse serum containing 100 µg/ml avidin (Sigma) for 30 minutes, and then incubated with saturated solution of biotin (Sigma) in TBS for an additional 15 minutes. This initial step has been shown to minimize any nonspecific reaction derived from avidin-biotin-peroxidase complex (ABC). The human antisera from AIDS patients or normal subjects were then used at 1:200 dilution followed by biotin-labelled goat anti-human immunoglobulin (Tago, Burlingame, California) at 1:200 dilutions and ABC (Vector Lab., Burlingame, California). Each incubation step was conducted for 30 minutes with extensive washing between steps. The color reaction was developed in $DAB-Ni-H_2O_2$ solution and counterstained with methyl green. Controls for the technique were performed by omitting the secondary antibody.

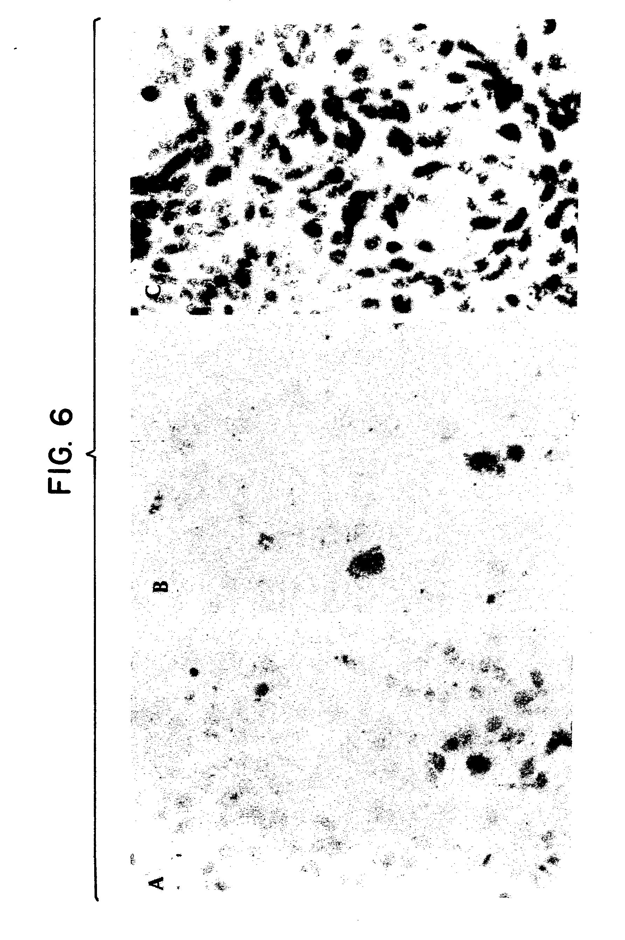

Sera of patients with AIDS produced positive immunochemical reactions with these infected cells, but not with normal NIH/3T3 cells (Figure 6(C) and 6(B), respectively). The reaction appeared to be positive in both nuclei and cytoplasm of Sb51 cells. However, many of the nuclei strained significantly stronger than the cytoplasm. A population of smaller round cells with apparently fewer cellular processes were found to be most heavily stained. Using this assay, 23 of 24 sera from AIDS patients, whether they presented with Kaposi's sarcoma, Kaposi's sarcoma with opportunistic infections, or opportunistic infections alone, were positive (Table 1). Serum from only one AIDS patient, with both Kaposi's sarcoma and opportunistic infections, showed weak positivity. Twenty-six of 30 non-AIDS normal human sera showed no reactivity to the infected Sb51 cells. One such negative reaction is shown in Figure 6(A). The other four sera showed mild reactivity to these cells. However, staining intensity was significantly less than that seen in the reactions of AIDS patients' sera.

-20-

## TABLE 1

Prevalence of Serum Antibodies to Sb51 Cells
in AIDS Patients with Various Clinical Presentations

| Subjects | Risk Group | | Total Number | Number Positive for Antibodies to SB51 Cells ** |
| --- | --- | --- | --- | --- |
| | Male Homo-sexual | Other | | |
| Patients w/AIDS * | 23 | 1 * | 24 | 23 |
| Kaposi's sarcoma | 8 | | 8 | 8 |
| Opportunistic infection | 5 | 1 * | 6 | 6 |
| Kaposi's sarcoma & opportunistic infections | 10 | | 10 | 9 |
| Normal Controls | | | 30 | 0 ** |

\*    Female, sexual partner of bisexual males.

\*\*   Four non-AIDS control sera showed mild
reactivity; all the other control sera did
not elicit any reaction.

Sera from patients with ARC were tested in the same manner.
All such sera tested positive.

## EXAMPLE 9

### Identification of Viral Infected
### Cells in Tissues of AIDS Patients

Lymph node, spleen, Kaposi's sarcoma and brain tissues from
AIDS patients were fixed in Formalin and processed in paraffin
sections. An immunoperoxidase assay, such as described in
Example 8, was performed using antisera from mice or rabbits
prepared as described in Example 6 in place of the antisera from AIDS patients. Virus-infected cells were identified in virtually all of the tissues examined. Electron microscopy was performed to confirm the infection by the virus of the invention. Viral nucleocapsids in the nuclei and the cytoplasm of cytopathic cells in these tissues were identified. Mature virions were also seen in some of the cells of the infected tissues.

## GENERAL DISCUSSION

In the dynamic process of viral infection, the first encounter of an infectious viral particle with the surface of a host cell initiates a complex series of events, the outcome of which determines whether or not successful penetrations and subsequent viral replications ensue. Howe, C., et al., Comprehensive Virology 16, 1 (1980). Direct introduction of an infectious agent's genetic material into cells which may or may not be normally susceptible to the corresponding whole virus, bypasses many components with which virions must interact in order to penetrate into the cytoplasm. Gajdusek et al., supra; Howe et al., supra. Genetic materials obtained from spleen and Kaposi's sarcoma tissues of two AIDS patients were found to contain transforming genetic elements. Phenotypically transformed foci were identified following transfection of this genetic material into normal NIH/3T3 cells. The malignant phenotype which was characterized by rapid cell growth and pile-up in multi-layers could similarly be transmitted in subsequent cycles of transfection. Careful examination of these transfectants (Sb51 and Kb43) revealed that the cells were persistently infected with a de novo virus. Only occasional lytic plaques were identified in the cultures. Prominent build-up of a large quantity of nucleocapsids in the nuclei of these persistently infected cells was often identified. There were also cytoplasmic inclusions of nucleocapsids with or without being membrane bound. Endoplasmic reticulum appeared to play an important role in viral maturation and/or assembly. Although virions could be identified in the nuclei, mature virions were usually seen in the cytoplasm or associated with the plasma membrane of disrupted cytolytic cells.

The virions were roughly spherical. However, heterogenecity in both particle shape and size was noted. The diameter of the pleomorphic particles varied between 140-280 nm. The thin nucleocapsids inside the virions measured about 8-9 nm. The more electron-dense and partially granular nucleocapsids organized in the nuclei and cytoplasm measured approximately 18 nm to 25 nm.

Reverse transcriptase activity was assayed using either cell lysates or culture supernatants of the viral infected cells, according to the method of Houts, G.E., et al., J.of Virology 29, 517 (1979). No nucleotide synthesis was detected. In addition, morophology, size, processes of assemblage and maturation of this virus do not suggest a retrovirus. Furthermore, the ultrastructural studies of this virus suggest that it belongs to no specific group of viruses in current classification. However, some of the replicative and maturation processes observed can be consistent with those of large DNA viruses like herpetic viruses. Roizman, B., et al., 3, 229-382 (1974). Using monoclonal antibodies (Biotech, Inc.) and immunoperoxidase techniques, the following viral antigens were tested and found negative in Sb51 cells: (a) Epstein-Barr Virus (EBV) early antigen D component, early antigen R component, membrane antigen and capsid antigen; (b) Cytomegalovirus (CMV) early and late antigens; (c) Herpes Simplex I and II (Cooper Biomed.). In addition, sera from two non-AIDS patients with documented CMV infections failed to interact with Sb51 cells.

The virus is pathogenic in experimental small animals. Infection of the virus caused an acute disease in 10 to 12 days. Although most of the signs of acute infection disappeared, the animals had significant morbidity as well as mortality in three months. More than 40% of the nude mice had died in this period. The animals examined in necropsy showed either lymphocyte depletion or systemic lymphadenopathy. Prominent plasmacytosis was noted in the animal with lymphadenopathy. All the histologic features were quite characteristic of those seen in human AIDS patients. Spindle cell proliferation in the subcutaneous tissue and the deep viscera-like liver was also produced in the experimental animals. The lesions were certainly consistent with the spindle cell tumors, Kaposi's sarcoma, in human AIDS patients. Viral nucleocapsids and mature virions could be identified in the lesions of the infected animals. The infection of this virus is believed to cause immune deficiency. Large skin infection with ulceration and salivary gland infection with abscess formation were commonly seen in these animals.

The immunodeficiency state becomes more evident in that some of the immunocompetent mice (Balb/c) were shown to be infected with P. carinii. The Balb/c mice appeared to be equally sensitive to the infection and expired in a significantly shorter experimental period. Consistent with the histologic evidence of lymphocyte depletion, the infected animals' immunologic functions were markedly suppressed. To document that the viral infection can be passed to other mice, some of the infected animals' spleens were homogenized and reintroduced into other mice. Those mice became infected with the virus.

By an immunocytochemical technique, sera from nearly all patients with AIDS (23 of 24) and all patients with ARC were shown to possess unique antibodies that recognized the newly acquired antigens in the viral infected cells, but not in the parent normal NIH/3T3 cells. The implications of this viral infection in patients with AIDS appear to be significant. By the same technique, viral antigens were shown in infected tissues of patients with AIDS using antibodies raised against the virus.

While the invention has been described in connection with specific embodiments thereof, it will be understood that it is capable of further modifications. This application is intended to cover any variations, uses or adaptations of the invention following, in general, the principles of the invention, and including such departures from the present disclosure as come within known and customary practice within the art to which the invention pertains.

CLAIMS

1. A virus isolatable from tissues of patients with AIDS, which is capable of transforming NIH/3T3 cells, capable of infecting nude mice and Balb/c mice to produce AIDS-like symptoms, and which has a density of 1.17 to 1.20 and a size range of 100 to 900 nm.

2. A virus isolatable from tissues of patients with AIDS, which has the characteristics of the virus produced by the cell line ATCC No. CRL 9127.

3. A virus isolatable from tissues of patients with AIDS, as produced by the cell line ATCC No. CRL 9127.

4. A process for isolating a virus according to any preceding claim, which comprises:

(a) obtaining tissue from a patient with AIDS,

(b) isolating genetic material from the tissue,

(c) transfecting NIH/3T3 cells with the genetic material, and

(d) isolating the virus from the infected NIH/3T3 cells.

5. A process for isolating a virus according to any of claims 1 to 3, which comprises:

(a) obtaining tissue from a patient with AIDS,

(b) co-cultivating the tissue with a recipient cell line or recipient cells,

(c) identifying transformed cells, and

(d) isolating the virus from the infected cells.

6. An antibody which specifically reacts with a virus according to any of claims 1 to 3.

7. A cell line which is persistently infected with a virus according to any of claims 1 to 3.

8. A cell line according to claim 7, derived from NIH/3T3 cells.

9. A cell line according to claim 8, identified as ATCC No. CRL 9127.

10. A method for the detection of antibodies which specifically bind to the antigenic sites of a virus according to any of claims 1 to 3 or contained in a cell line according to any of claims 7 to 9, in a sample of the body fluid of a patient with AIDS or ARC, which comprises contacting the virus with the sample, and measuring the formation of antigen-antibody complex.

11. A method for the detection of viral antigens of a virus according to any of claims 1 to 3, in a sample of infected tissue of a patient with AIDS or ARC, which comprises reacting an antibody specific to the virus with the sample, and measuring the formation of antigen-antibody complex.

12. A diagnostic kit for the detection of AIDS-specific antibodies, which comprises a container, means for detecting an antigen-antibody complex, and a virus according to any of claims 1 to 3 or a cell line according to any of claims 7 to 9.

13. A diagnosit kit for the detection of viral antigens of a virus according to any of claims 1 to 3 in tissues of patients with AIDS or ARC, which comprises a container, an antibody according to claim 6, and means for detecting an antigen-antibody complex.

14. A vaccine composition comprising a virus according to any of claims 1 to 3 which has been inactivated, and a physiologically-acceptable carrier.

15. A vaccine composition comprising a viral antigen of a virus according to any of claims 1 to 3, and a physiologically-acceptable carrier.

European Patent Office

0250128

Application number:

87304968.4

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:

CRL 9127

# FIG. I

0250128

FIG. 2

FIG. 3

4/6

FIG. 4

5/6

# FIG. 5

A

B

C

# FIG. 6